# EUROPÄISCHE PATENTSCHRIFT

(11) **EP 0 512 346 B1**
(45) Veröffentlichungstag und Bekanntmachung des Hinweises auf die Patenterteilung: **28.06.1995**
(21) Anmeldenummer: 92107127.0
(22) Anmeldetag: 27.04.1992
(51) Int. Cl.: C07C 51/41, C08F 36/00, C07C 53/128, C07C 61/08

(54) **Verfahren zur Herstellung von Metallcarboxylaten sowie deren Verwendung für die Polymerisation von für eine Ziegler-Natta-Polymerisation geeigneten Monomeren**
Process for preparing metal carboxylates and their use in the polymerisation of monomers suitable for the Ziegler-Natta polymerisation
Procédé de préparation de carboxylates de métaux ainsi que leur utilisation pour la polymerisation des monomères d'après le procédé Ziegler-Natta

(30) Priorität: 08.05.1991 DE 4115034
(43) Veröffentlichungstag der Anmeldung: 11.11.1992
(73) Patentinhaber: BAYER AG, 51368 Leverkusen (DE)
(72) Erfinder: Knauf, Thomas, Dr., c/o Polysar Rubber Corporation, Sarnia, Ontario N7T 7M2 (CA); Obrecht, Werner, Dr., W-4130 Moers 2 (DE)

(56) Entgegenhaltungen:
- CH-A- 313 372
- DE-B- 1 016 389
- GB-A- 2 140 435
- US-A- 2 423 619
- US-A- 3 162 660

## Beschreibung

Die Erfindung betrifft ein Verfahren zur Herstellung von Metallcarboxylaten, insbesondere von Carboxylaten der Übergangsmetalle, der Lanthaniden und Aktiniden, sowie die Verwendung der Metallcarboxylate für die Polymerisation von Monomeren, insbesondere von Dienen, die für eine Ziegler-Natta-Polymerisation geeignet sind.

Die Herstellung von Metallcarboxylaten der genannten Art, insbesondere von Carboxylaten der seltenen Erden, wie Neodymcarboxylaten, ist bekannt (siehe z.B. GB 2 140 435 und JP 61/176 554).

So ist z.B. aus GB 2 140 435 bekannt, daß die Umsetzung von wäßriger Neodymchloridlösung mit drei Äquivalenten wäßriger Natriumversatatlösung zum gewünschten Neodymversatat führt. Die Reinigung von Neodymverstat erfolgt mittels Extraktion mit Toluol und anschließender Filtration der toluolischen Neodymverstatlösung.

Weiterhin ist aus JP 61/176Z554 bekannt, daß Neodymoctanoat ausgehend von Neodymacetat und Natriumoctanoat hergestellt werden kann. Das Neodymacetat wird aus Neodymchlorid und einem Überschuß Essigsäure hergestellt. Neodymoctanoat wird nach der Herstellung durch Umkristallisation gereinigt.

Nachteilig bei diesen bekannten Verfahren ist z.B. die Verwendung von NaOH als Base. Im Überschuß führt sie zur teilweisen Zersetzung von Neodymcarboxylat. Außerdem verbleibt Natrium im Produkt. Weiterhin beinhalten die obigen Verfahren zwei (GB 2 140 435) bzw. drei (JP 61/176 554) separate Reaktionsschritte, die einen großen apparativen Aufwand bedeuten (mehrere Kessel, Dosiervorrichtungen etc). Bei beiden Verfahren fallen Feststoffe an, die extrahiert und filtriert werden müssen und wobei das Lösungsmittel auszutauschen ist (GB 2 140 435) bzw. wonach umkristallisiert werden muß (JP 61/176 554). Daraus resultieren beträchtliche Ausbeuteverluste.

Es wurde nun gefunden, daß man die oben geschilderten Nachteile bei der Herstellung von Metallcarboxylaten vermeiden kann, wenn man die den Carboxylaten zugrundeliegenden organischen Carbonsäuren mit 2 bis 20 C-Atomen mit Ammoniak und/oder Aminen und/oder Tetraalkylammoniumhydroxyden und den entsprechenden Metallnitraten in Gegenwart von inerten, organischen Lösungsmitteln bei Temperaturen von 0 bis 150°C umsetzt.

Das erfindungsgemäße Verfahren kann durch folgende allgemeine Formel dargestellt werden:

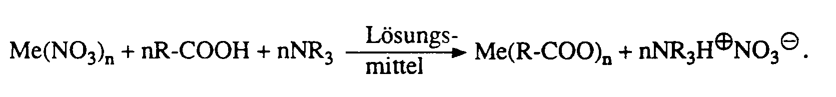

Als Metallnitrate werden bei dem erfindungsgemäßen Verfahren insbesondere die Nitrate der Lanthaniden, der Übergangsmetalle der dritten, vierten und achten Nebengruppe des Periodensystems und der Aktiniden eingesetzt. Genannt werden beispielsweise die Nitrate des Eisens, Kobalts, Nickels, Cers, Praseodyms, Neodyms, Samariums, Europiums, Gadoliniums, Terbiums, Dysprosiums, Holmiums, Erbiums, Thuliums, Ytterbiums, Lutetiums, Thoriums, Scandiums, Ytteriums, Lanthans und Titans, bevorzugt die Nitrate des Cers, Praseodyms, Neodyms, Kobalts und des Nickels. Selbstverständlich können die Metallnitrate im Gemisch untereinander eingesetzt werden. Dabei kann das Mischungsverhältnis beliebig gewählt werden. Dieses richtet sich üblicherweise nach dem späteren Verwendungszweck des Metallcarboxylats.

Als organische Carbonsäuren kommen aliphatische und cycloaliphatische Carbonsäuren in Frage. Bevorzugt werden solche mit 2 bis 18 C-Atomen eingesetzt. Besonders bevorzugt sind organische Carbonsäuren der Formel 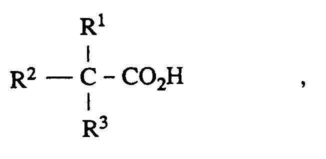
worin
- R¹, R² und R³: gleich oder verschieden sind und für Wasserstoff oder Alkylreste mit 1 bis 10 Kohlenstoffatomen stehen, wobei die Summe der Kohlenstoffatome von R¹, R² und R³ 0 bis 16 C-Atome beträgt.

Weiterhin kommen aromatische, organische Carbonsäuren in Frage. Bevorzugt werden solche mit 7 bis 19 C-Atomen eingesetzt. Besonders bevorzugt sind aromatische, organische Carbonsäuren der Formel

R₄-CO₂H,

worin
- R₄: für einen Arylrest oder einen Alkylarylrest mit 6 bis 17 Kohlenstoffatomen steht.

Als Beispiele seien folgende Carbonsäuren genannt: Essigsäure, Propionsäure, n-Buttersäure, iso-Buttersäure, Valeriansäure, Pivalinsäure, Hexancarbonsäure, Hexancarbonsäure-2, Cyclohexancarbonsäure, 3-Cyclohexylpropionsäure, 2-Ethylhexancarbonsäure, 2-Methyl-2-ethyl-pentansäure, 2,2-Diethyl-pentansäure, 2,2-Dimethyl-hexansäure, 2-Methyl-2-ethyl-hexansäure, 2,2-Diethyl-hexansäure, 2-Ethyl-2-propyl-hexansäure, 2-Ethyl-2-butyl-heptansäure, 2,2-Diethyl-heptansäure, 2,2-Diethyl-octansäure und 2-Methyl-2-butyl-octansäure, Heptansäure-1, Octancarbonsäure-1, Nonancarbonsäure-1, Versaticsäure (Handelsprodukt der Shell Chemie), Laurinsäure, Myristinsäure, Palmitinsäure, Stearinsäure, Ölsäure, Benzoesäure, Naphthalinsäure, Phenylessigsäure, Triphenylessigsäure, Tricyclohexylessigsäure, bevorzugt Pivalinsäure, Cyclohexancarbonsäure und 2-Ethylhexancarbonsäure.

Die genannten organischen Carbonsäuren können sowohl einzeln als auch im Gemisch untereinander eingesetzt werden. Das günstigste Mischungsverhältnis läßt sich leicht durch geeignete Vorversuche bestimmen.

Als Amine werden in das erfindungsgemäße Verfahren solche der Formel

NR₃ ,

worin
- R: gleich oder verschieden ist und für Methyl, Ethyl, Propyl, Butyl oder Pentyl sowie für deren Isomeren steht, eingesetzt.

Als Tetraalkylammoniumhydroxide werden in das erfindungsgemäße Verfahren solche der Formel

NR₄OH,

worin
- R: gleich oder verschieden ist und für Methyl, Ethyl, Propyl, Butyl, Pentyl, Hexyl, Heptyl, Octyl oder Benzyl sowie für deren entsprechenden Isomeren steht, eingesetzt.

Sowohl das Ammoniak als auch die Amine können in gasförmiger, flüssiger oder in wäßriger Lösung (10 bis 30 %ig) eingesetzt werden. Die Tetraalkylammoniumhydroxide werden bevorzugt als wäßrige Lösung (20 bis 80 %ig) eingesetzt.

Das erfindungsgemäße Verfahren wird bevorzugt bei Temperaturen von 20 bis 40°C durchgeführt. Dabei ist es möglich, das Verfahren ohne Druck als auch unter Druck (1 bis 50 bar) durchzuführen.

Als inerte, organische Lösungsmittel eignen sich für das erfindungsgemäße Verfahren solche, die üblicherweise für die Ziegler-Natta-Polymerisation geeignet sind. Geeignete inerte, organische Lösungsmittel sind beispielsweise aliphatische, cycloaliphatische oder aromatische Kohlenwasserstoffe mit 4 bis 20 Kohlenstoffatomen, bevorzugt 4 bis 10 Kohlenstoffatomen. Genannt seien Butan, Pentan, Hexan, Heptan, Octan, Cyclohexan, Cyclodecan und Toluol sowie deren entsprechenden Isomeren, bevorzugt n-Hexan und Cyclohexan.

Die geeignetsten inerten organischen Lösungsmittel für das erfindungsgemäße Verfahren können leicht durch entsprechende Vorversuche ermittelt werden. Die inerten organischen Lösungsmittel können sowohl einzeln als auch im Gemisch untereinander eingesetzt werden.

Nach dem erfindungsgemäßen Verfahren setzt man üblicherweise pro Mol Metallnitrat etwa 2 bis 6 Mol, bevorzugt 2 bis 4 Mol, organische Carbonsäure ein.

Das Molverhältnis von einzusetzendem Metallnitrat zum Ammoniak und/oder Amin und/oder Tetraalkylammoniumhydroxid beträgt in Abhängigkeit von der Wertigkeit des zugrundeliegenden Metalls 1:2 bis 10 (zweiwertig) oder 1:3 bis 15 (dreiwertig) oder 1:4 bis 20 (vierwertig).

Für das erfindungsgemäße Verfahren hat es sich als vorteilhaft erwiesen, wenn man die Menge des inerten, organischen Lösungsmittels so bemißt, daß sich Konzentrationen des erhaltenen Metallcarboxylats im Lösungsmittel von etwa 0,1 bis 80 Gew.-%, bevorzugt 5 bis 60 Gew.-%, ergeben.

Das erfindungsgemäße Verfahren kann beispielsweise so durchgeführt werden, daß man die entsprechenden Mengen an inertem, organischem Lösungsmittel, Metallnitrat und an organischer Carbonsäure vorlegt und anschließend die entsprechende Menge an Ammoniak und/oder Amin und/oder Tetraalkylammoniumhydroxid unter kräftigem Rühren bei den zuvor angegebenen Temperaturen eindosiert Nach Ende der exothermen Reaktion wird die entstehende wäßrige Phase abgetrennt und die organische Phase mehrmals mit Wasser extrahiert. Zur Gewinnung der Metallcarboxylate wird das Wasser anschließend mittels einer Azeotropdestillation abdestilliert.

Metallcarboxylate, die in den oben aufgeführten Lösungsmitteln, unlöslich sind, werden mehrmals mit Wasser gewaschen und (im Trockenschrank bei ca. 80°C und 16 Torr) getrocknet.

Ein weiterer Gegenstand der Erfindung ist die Verwendung der nach dem erfindungsgemäßen Verfahren hergestellten Metallcarboxylate zur Polymerisation von Monomeren, die für die Ziegler-Natta-Polymerisation geeignet sind.

Als solche Monomere kommen in Frage insbesondere Diene, wie Butadien, Isopren, 2-Phenylbutadien, 2,3-Dimethylbutadien, 1,3-Hexadien und 1,3-Octadien.

Die Polymerisation von solchen Monomeren ist bekannt und wird beispielsweise beschrieben von John Boor, Jr., Ziegler-Natta Catalysts and Polymerizations, Academic Press, New York, San Francisco, London 1979.

Besonders geeignet sind die erfindungsgemäßen Metallcarboxylate, insbesondere die Carboxylate der seltenen Erden für die Polymerisation von konjugierten Dienen (siehe z.B. EP 007 027, DE 3 224 288, EP 011 184, EP 0 201 979, EP 0 201 962, EP 0 127 236, EP 0 207 558).

Bei der Polymerisation der Diene entstehen wertvolle Elastomere, die wiederum z.B. für die Herstellung von Reifen, Golfbällen und anderen Gummiwaren geeignet sind.

### Beispiele

A. In einem 4 l-Kolben werden 0,418 mol wäßrige Neodymnitratlösung und 1,317 mol Versaticsäure in 1665 ml Cyclohexan vorgelegt. Anschließend werden bei Raumtemperatur 1,267 mol Ammoniak unter kräftigem Rühren zugegeben. Nach Beendigung der Reaktion wird 2x mit 100 ml dest. Wasser extrahiert. Abschließend wird mittels azeotroper Destillation restliches Wasser aus der 17,5 %igen, violetten Neodymversatatlösung in Cyclohexan entfernt.
B. In einem 1 l-Kolben werden 0,40 mol 2-Ethylhexancarbonsäure bei Raumtemperatur unter Rühren mit 0,40 mol Triethylamin versetzt. Nach Abklingen der exothermen Reaktion wird mit 65 ml Cyclohexan verdünnt und 0,20 mol Cobaltnitrat zugegeben. Es wird 30 min. nachgeführt. Anschließend wird die Reaktionslösung filtriert. Es verbleibt eine 55,5 %ige, blaue Cobaltoctoatlösung in Cyclohexan.
C. 0,10 mol wäßrige Neodymnitratlösung, 0,30 mol Versaticsäure und 1606 ml Cyclohexan werden in einem 4 l-Kolben vorgelegt. Dann wird bei Raumtemperatur 0,303 mol wäßrige Tetrabutylammoniumhydroxidlösung (40 %ig) unter kräftigem Rühren zugegeben. Nach Beendigung der exothermen Reaktion wird die organische Phase 5x mit 200 ml Wasser extrahiert. Abschließend wird aus der cyclohexanischen Neodymversatatlösung durch azeotrope Destillation Wasser entfernt.
D. Eine Emulsion von 0,30 mol Pivalinsäure in 0,10 mol wäßriger Neodymnitratlösung wird bei Raumtemperatur unter kräftigem Rühren mit 0,303 mol wäßriger Ammoniaklösung (25 %ig) versetzt. Es tritt eine exotherme Reaktion ein und ein rosafarbender Niederschlag fällt aus. Der Niederschlag wird abzentrifugiert und mehrmals mit Wasser gewaschen. Nach 15 Stunden Trocknen im Vakuum bei 80°C verbleiben 41,8 g Neodympivalat.
E. Eine Emulsion von 0,10 mol wäßriger Neodymnitratlösung, 0,1575 mol Cyclohexancarbonsäure und 0,1575 mol Versaticsäure in 1050 ml Cyclohexan wird unter kräftigem Rühren bei Raumtemperatur mit 0,32 mol Ammoniak versetzt. Nach Abklingen der exothermen Reaktion wird 2x mit 100 ml Wasser extrahiert. Die violette Neodymcarboxylatlösung wird durch azeotrope Destillation getrocknet.

## Patentansprüche (Patentansprüche für folgende(n) Vertragsstaat(en): DE, FR, GB, IT)

1. Verfahren zur Herstellung von Metallcarboxylaten, dadurch gekennzeichnet, daß man die den Carboxylaten zugrundeliegenden organischen Carbonsäuren mit 2 bis 20 C-Atomen mit Ammoniak und/oder Aminen und/oder Tetraalkylammoniumhydroxiden und den entsprechenden Metallnitraten in Gegenwart von inerten, organischen Lösungsmitteln bei Temperaturen von 0 bis 150°C umsetzt.

2. Verfahren nach Anspruch 1, dadurch gekennzeichnet, daß man als organische Carbonsäuren solche der Formel 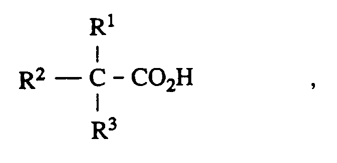 worin
R¹, R² und R³ gleich oder verschieden sind und Wasserstoff oder Alkylreste mit 1 bis 10 Kohlenstoffatomen darstellen, wobei die Summe der Kohlenstoffatome von R¹, R² und R³ 0 bis 16 C-Atome beträgt,
oder solche der Formel
R₄-CO₂H,
worin
R⁴ für einen Arylrest oder einen Alkylarylrest mit 6 bis 17 Kohlenstoffatomen steht,
einsetzt.

3. Verfahren nach Ansprüchen 1 und 2, dadurch gekennzeichnet, daß man als Amine solche der Formel
NR₃ ,
worin
R gleich oder verschieden ist und für Methyl, Ethyl, Propyl, Butyl, Pentyl oder deren Isomeren steht,
einsetzt.

4. Verfahren nach Ansprüchen 1 und 2, dadurchgekennzeichnet, daß man als Tetraalkylammoniumhydroxide solche der Formel
NR₄OH,
worin
R gleich oder verschieden ist und für Methyl, Ethyl, Propyl, Butyl, Pentyl, Hexyl, Octyl, Benzyl oder deren entsprechenden Isoemren steht,
einsetzt.

5. Verfahren nach Ansprüchen 1 bis 4, dadurch gekennzeichnet, daß man das Ammoniak und/oder die Amine und/oder die Tetraalkylammoniumhydroxide in wäßriger Lösung einsetzt.

6. Verfahren nach Ansprüchen 1 bis 5, dadurch gekennzeichnet, daß man als Metallnitrate solche der Übergangsmetalle der dritten, vierten und achten Nebengruppe des Periodensystems, der Lanthaniden oder der Aktiniden einsetzt.

7. Verfahren nach Ansprüchen 1 bis 6, dadurch gekennzeichnet, daß man als inerte, organische Lösungsmittel aliphatische, cycloaliphatische und/oder aromatische Kohlenwasserstoffe mit 4 bis 20 Kohlenstoffatomen einsetzt.

8. Verfahren nach Ansprüchen 1 bis 7, dadurch gekennzeichnet, daß man pro Mol Metallnitrat 2 bis 6 Mol organische Carbonsäure einsetzt.

9. Verfahren nach Ansprüchen 1 bis 8, dadurch gekennzeichnet, daß man pro Mol Metallnitrat 2 bis 20 Mol an Ammoniak und/oder Aminen und/oder Tetraalkylammoniumhydroxiden einsetzt.

10. Verwendung der nach dem Verfahren des Anspruchs 1 hergestellten Metallcarboxylaten zur Polymerisation von Monomeren, die für die Ziegler-Natta-Polymerisation geeignet sind.

## Patentansprüche (Patentansprüche für folgende(n) Vertragsstaat(en): ES)

1. Verfahren zur Herstellung von Metallcarboxylaten, dadurch gekennzeichnet, daß man die den Carboxylaten zugrundeliegenden organischen Carbonsäuren mit 2 bis 20 C-Atomen mit Ammoniak und/oder Aminen und/oder Tetraalkylammoniumhydroxiden und den entsprechenden Metallnitraten in Gegenwart von inerten, organischen Lösungsmitteln bei Temperaturen von 0 bis 150°C umsetzt.

2. Verfahren nach Anspruch 1, dadurch gekennzeichnet, daß man als organische Carbonsäuren solche der Formel 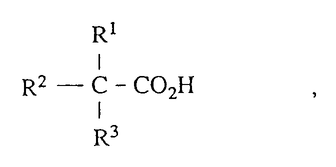 worin
R¹, R² und R³ gleich oder verschieden sind und Wasserstoff oder Alkylreste mit 1 bis 10 Kohlenstoffatomen darstellen, wobei die Summe der Kohlenstoffatome von R¹, R² und R³ 0 bis 16 C-Atome beträgt,
oder solche der Formel
R₄-CO₂H,
worin
R⁴ für einen Arylrest oder einen Alkylarylrest mit 6 bis 17 Kohlenstoffatomen steht,
einsetzt.

3. Verfahren nach Ansprüchen 1 und 2, dadurch gekennzeichnet, daß man als Amine solche der Formel
NR₃ ,
worin
R gleich oder verschieden ist und für Methyl, Ethyl, Propyl, Butyl, Pentyl oder deren Isomeren steht,
einsetzt.

4. Verfahren nach Ansprüchen 1 und 2, dadurchgekennzeichnet, daß man als Tetraalkylammoniumhydroxide solche der Formel
NR₄OH,
worin
R gleich oder verschieden ist und für Methyl, Ethyl, Propyl, Butyl, Pentyl, Hexyl, Octyl, Benzyl oder deren entsprechenden Isoemren steht,
einsetzt.

5. Verfahren nach Ansprüchen 1 bis 4, dadurch gekennzeichnet, daß man das Ammoniak und/oder die Amine und/oder die Tetraalkylammoniumhydroxide in wäßriger Lösung einsetzt.

6. Verfahren nach Ansprüchen 1 bis 5, dadurch gekennzeichnet, daß man als Metallnitrate solche der Übergangsmetalle der dritten, vierten und achten Nebengruppe des Periodensystems, der Lanthaniden oder der Aktiniden einsetzt.

7. Verfahren nach Ansprüchen 1 bis 6, dadurch gekennzeichnet, daß man als inerte, organische Lösungsmittel aliphatische, cycloaliphatische und/oder aromatische Kohlenwasserstoffe mit 4 bis 20 Kohlenstoffatomen einsetzt.

8. Verfahren nach Ansprüchen 1 bis 7, dadurch gekennzeichnet, daß man pro Mol Metallnitrat 2 bis 6 Mol organische Carbonsäure einsetzt.

9. Verfahren nach Ansprüchen 1 bis 8, dadurch gekennzeichnet, daß man pro Mol Metallnitrat 2 bis 20 Mol an Ammoniak und/oder Aminen und/oder Tetraalkylammoniumhydroxiden einsetzt.

## Claims (Claims for the following Contracting State(s): DE, FR, GB, IT)

1. A process for the production of metal carboxylates, characterized in that the C₂₋₂₀ carboxylic acids on which the carboxylates are based are reacted with ammonia and/or amines and/or tetraalkyl ammonium hydroxides and the corresponding metal nitrates in the presence of inert organic solvents at temperatures of 0 to 150°C.

2. A process as claimed in claim 1, characterized in that the organic carboxylic acids used correspond to the following formula: 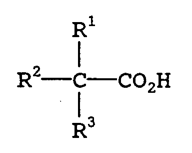 in which
R¹, R² and R³ may be the same or different and represent hydrogen or C₁₋₁₀ alkyl radicals, the sum of the carbon atoms of R¹, R² and R³ being from 0 to 16 C atoms,
or to the following formula:
R₄-CO₂H
in which
R₄ is an aryl radical or a C₆₋₁₇ alkylaryl radical.

3. A process as claimed in claims 1 and 2, characterized in that the amines used correspond to the following formula
NR₃
in which
the R's may be the same or different and represent methyl, ethyl, propyl, butyl or pentyl and their isomers.

4. A process as claimed in claims 1 and 2, characterized in that the tetraalkyl ammonium hydroxides used correspond to the following formula:
NR₄OH
in which
the R's may be the same or different and represent methyl, ethyl, propyl, butyl, pentyl, hexyl, heptyl, octyl or benzyl or their corresponding isomers.

5. A process as claimed in claims 1 to 4, characterized in that the ammonia and/or the amines and/or the tetraalkyl ammonium hydroxides are used in aqueous solution.

6. A process as claimed in claims 1 to 5, characterized in that the metal nitrates used are those of the transition metals of the 3rd, 4th and 8th secondary group of the periodic system, the lanthanides or actinides.

7. A process as claimed in claims 1 to 6, characterized in that aliphatic, cycloaliphatic and/or aromatic hydrocarbons containing 4 to 20 carbon atoms are used as the inert organic solvents.

8. A process as claimed in claims 1 to 7, characterized in that 2 to 6 moles of organic carboxylic acid are used per mole of metal nitrate.

9. A process as claimed in claims 1 to 8, characterized in that 2 to 20 moles of ammonia and/or amines and/or tetraalkyl ammonium hydroxides are used per mole of metal nitrate.

10. The use of the metal carboxylates produced by the process claimed in claim 1 for the polymerization of monomers suitable for Ziegler-Natta polymerization.

## Claims (Claims for the following Contracting State(s): ES)

1. A process for the production of metal carboxylates, characterized in that the C₂₋₂₀ carboxylic acids on which the carboxylates are based are reacted with ammonia and/or amines and/or tetraalkyl ammonium hydroxides and the corresponding metal nitrates in the presence of inert organic solvents at temperatures of 0 to 150°C.

2. A process as claimed in claim 1, characterized in that the organic carboxylic acids used correspond to the following formula: 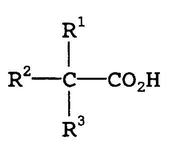 in which
R¹, R² and R³ may be the same or different and represent hydrogen or C₁₋₁₀ alkyl radicals, the sum of the carbon atoms of R¹, R² and R³ being from 0 to 16 C atoms,
or to the following formula:
R₄-CO₂H
in which
R₄ is an aryl radical or a C₆₋₁₇ alkylaryl radical.

3. A process as claimed in claims 1 and 2, characterized in that the amines used correspond to the following formula
NR₃
in which
the R's may be the same or different and represent methyl, ethyl, propyl, butyl or pentyl and their isomers.

4. A process as claimed in claims 1 and 2, characterized in that the tetraalkyl ammonium hydroxides used correspond to the following formula:
NR₄OH
in which
the R's may be the same or different and represent methyl, ethyl, propyl, butyl, pentyl, hexyl, heptyl, octyl or benzyl or their corresponding isomers.

5. A process as claimed in claims 1 to 4, characterized in that the ammonia and/or the amines and/or the tetraalkyl ammonium hydroxides are used in aqueous solution.

6. A process as claimed in claims 1 to 5, characterized in that the metal nitrates used are those of the transition metals of the 3rd, 4th and 8th secondary group of the periodic system, the lanthanides or actinides.

7. A process as claimed in claims 1 to 6, characterized in that aliphatic, cycloaliphatic and/or aromatic hydrocarbons containing 4 to 20 carbon atoms are used as the inert organic solvents.

8. A process as claimed in claims 1 to 7, characterized in that 2 to 6 moles of organic carboxylic acid are used per mole of metal nitrate.

9. A process as claimed in claims 1 to 8, characterized in that 2 to 20 moles of ammonia and/or amines and/or tetraalkyl ammonium hydroxides are used per mole of metal nitrate.

## Revendications (Revendications pour l'(les) Etat(s) contractant(s) suivant(s): DE, FR, GB, IT)

1. Procédé de préparation de carboxylates métalliques, caractérisé en ce que l'on fait réagir les acides organiques carboxyliques en C₂-C₂₀ correspondant aux carboxylates avec l'ammoniac et/ou des amines et/ou des hydroxydes de tétraalkylammonium et les nitrates métalliques correspondants en présence de solvants organiques inertes à des températures de 0 à 150°C.

2. Procédé selon la revendication 1, caractérisé en ce que l'on met en oeuvre en tant qu'acides organiques carboxyliques des acides de formule 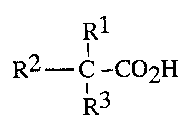 dans laquelle R¹, R² et R³, ayant des significations identiques ou différentes, représentent chacun l'hydrogène ou un groupe alkyle en C₁-C₁₀, la somme des atomes de carbone de R¹, R² et R³ allant de 0 à 16, ou de formule
R₄-CO₂H
dans laquelle R₄ représente un groupe aryle ou alkylaryle en C₆-C₁₇.

3. Procédé selon les revendications 1 et 2, caractérisé en ce que l'on utilise en tant qu'amines des amines de formule
NR₃,
dans laquelle les symboles R, ayant des significations identiques ou différentes, représentent chacun un groupe méthyle, éthyle, propyle, butyle, pentyle ou leurs isomères.

4. Procédé selon les revendications 1 et 2, caractérisé en ce que l'on utilise en tant qu'hydroxydes de tétraalkylammonium, des composés de formule
NR₄OH,
dans laquelle les symboles R, ayant des significations identiques ou différentes, représentent chacun un groupe méthyle, éthyle, propyle, butyle, pentyle, hexyle, octyle, benzyle ou leurs isomères.

5. Procédé selon les revendications 1 à 4, caractérisé en ce que l'on met en oeuvre l'ammoniac et/ou les amines et/ou les hydroxydes de tétraalkylammonium à l'état de solutions aqueuses.

6. Procédé selon les revendications 1 à 5, caractérisé en ce que les nitrates métalliques mis en oeuvre sont les nitrates des métaux de transition du troisième, du quatrième et du huitième sous-groupes de la classification périodique, des lanthanides ou des actinides.

7. Procédé selon les revendications 1 à 6, caractérisé en ce que l'on utilise en tant que solvants organiques inertes des hydrocarbures aliphatiques, cycloaliphatiques et/ou aromatiques en C₄-C₂₀.

8. Procédé selon les revendications 1 à 7, caractérisé en ce que l'on met en oeuvre de 2 à 6 moles d'acide organique carboxylique par mole de nitrate métallique.

9. Procédé selon les revendications 1 à 8, caractérisé en ce que l'on met en oeuvre de 2 à 20 moles d'ammoniac et/ou d'amines et/ou d'hydroxydes de tétraalkylammonium par mole de nitrate métallique.

10. Utilisation des carboxylates métalliques préparés par le procédé selon la revendiction 1, pour la polymérisation de monomères aptes à une polymérisation de Ziegler-Natta.

## Revendications (Revendications pour l'(les) Etat(s) contractant(s) suivant(s): ES)

1. Procédé de préparation de carboxylates métalliques, caractérisé a ce que l'on fait réagir les acides organiques carboxyliques en C₂-C₂₀ correspondant aux carboxylates avec l'ammoniac et/ou des amines et/ou des hydroxydes de tétraalkylammonium et les nitrates métalliques correspondants en présence de solvants organiques inertes à des températures de 0 à 150°C.

2. Procédé selon la revendication 1, caractérisé en ce que l'on met en oeuvre en tant qu'acides organiques carboxyliques des acides de formule 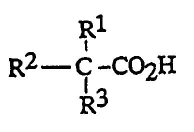 dans laquelle R¹, R² et R³, ayant des significations identiques ou différentes, représentent chacun l'hydrogène ou un groupe alkyle en C₁-C₁₀, la somme des atomes de carbone de R¹, R² et R³ allant de 0 à 16,
ou de formule
R₄-CO₂H
dans laquelle R₄ représente un groupe aryle ou alkylaryle C₆-C₁₇.

3. Procédé selon les revendications 1 et 2, caractérisé en ce que l'on utilise en tant qu'amines des amines de formule
NR₃,
dans laquelle les symboles R, ayant des significations identiques ou différentes, représentent chacun un groupe méthyle, éthyle, propyle, butyle, pentyle ou leurs isomères.

4. Procédé selon les revendications 1 et 2, caractérisé en ce que l'on utilise en tant qu'hydroxydes de tétralkylammonium, des composés de formule
NR₄OH,
dans laquelle les symboles R, ayant des significations identiques ou différentes, représentent chacun un groupe méthyle, éthyle, propyle, butyle, pentyle, hexyle, octyle, benzyle ou leurs isomères.

5. Procédé selon les revendications 1 à 4, caractérisé en ce que l'on met en oeuvre l'ammoniac et/ou les amines et/ou les hydroxydes de tétraalkylammonium à l'état de solutions aqueuses.

6. Procédé selon les revendications 1 à 5, caractérisé en ce que les nitrates métalliques mis en oeuvre sont les nitrates des métaux de transition du troisième, du quatrième et du huitième sous-groupes de la classification périodique, des lanthanides ou des actinides.

7. Procédé selon les revendications 1 à 6, caractérisé en ce que l'on utilise en tant que solvants organiques inertes des hydrocarbures aliphatiques, cycloaliphatiques et/ou aromatiques en C₄-C₂₀.

8. Procédé selon les revendications 1 à 7, caractérisé en ce que l'on met en oeuvre de 2 à 6 moles d'acide organique carboxylique par mole de nitrate métallique.

9. Procédé selon les revendications 1 à 8, caractérisé en ce que l'on met en oeuvre de 2 à 20 moles d'ammoniac et/ou d'amines et/ou d'hydroxydes de tétraalkylammonium par mole de nitrate métallique.
